# EUROPEAN PATENT APPLICATION

(11) **EP 3 698 737 A1**
(43) Date of publication of application: **26.08.2020**
(21) Application number: 20158861.3
(22) Date of filing: 21.02.2020
(51) Int. Cl.: A61B 17/32, A61B 17/00

(54) **RECIPROCATING SURGICAL SHAVER**

(30) Priority: 22.02.2019 US 201916282505; 27.02.2019 US 201916286748; 27.02.2019 US 201916287329; 01.03.2019 US 201916290047
(71) Applicant: Gyrus ACMI, Inc. D.B.A. Olympus Surgical Technologies America, Southborough MA 01772 (US)
(72) Inventor: ALSAFFAR, Ahmad, Southborough, MA 01772 (US); EDWARDS, Kevin, Southborough, MA 01772 (US); WILLHITE, Joel, Southboriugh, MA 01772 (US); CURCH, David, Southborough, MA 01772 (US); GOLDBERG, Daniel, Southborough, MA 01772 (US)
(74) Representative: Noack, Andreas

(57) **Abstract**

Disclosed herein is a medical device. The medical device includes a blade tube section (16), a blade drive system (28), and a mechanical arrangement. The blade tube section (16) includes an outer blade tube (24), an inner blade tube (26), and a cutting window (46) at a distal end (40) of the blade tube section (16). The mechanical arrangement is between the inner blade tube (26) and the blade drive system (28).

## Description

### BACKGROUND

### Field of the Invention

The invention relates to a medical device and more specifically relates to methods of reciprocation for a surgical shaver device.

### Brief Description of Prior Developments

Conventional surgical shavers generally use a rotational motor coupled with a parallel gear train to impart oscillatory motion on the shaver blades. However, using the oscillating motion to cut can tear and strip mucosa. Reciprocating blades, on the other hand, can create cleaner, more precise cuts.

Reciprocating surgical shavers exist in the market. However, these devices generally use air pressure from a vacuum to drive the reciprocation, which can result in a weak cutting stroke and in turn make the device unable to cut through the tissue necessary to complete a procedure.

Accordingly, there is a need to provide improved and reliable medical device configurations having reciprocating blades.

### SUMMARY

In accordance with one aspect of the invention, a medical device is disclosed. The medical device includes a blade tube section, a blade drive system, and a mechanical arrangement. The blade tube section includes an outer blade tube, an inner blade tube, and a cutting window at a distal end of the blade tube section. The mechanical arrangement is between the inner blade tube and the blade drive system.

### BRIEF DESCRIPTION OF THE DRAWINGS

The foregoing aspects and other features of the invention are explained in the following description, taken in connection with the accompanying drawings, wherein:
Fig. 1 is a side view of a medical device incorporating features of the invention;
Fig. 2 is an alternate embodiment of a cutting tip used in the medical device;
Fig. 3 is an alternate embodiment of a cutting tip used in the medical device;
Fig. 4 is an alternate embodiment of a cutting tip used in the medical device;
Fig. 5 is an alternate embodiment of a cutting tip used in the medical device;
Fig. 6 is a side view of a blade drive system used in the medical device;
Fig. 7 is a side view of an alternate blade drive system used in the medical device;
Fig. 8 is a top view of a blade drive system used in the medical device;
Fig. 9 is another top view of the blade drive system;
Fig. 10 is a perspective view of an alternate blade drive system used in the medical device;
Fig. 11 is a top view of a blade drive system used in the medical device;
Fig. 12 is a partial view of the blade drive system;
Fig. 13 is a perspective view of the blade drive system;
Fig. 14 is a top view of a blade drive system used in the medical device;
Fig. 15 is another top view of the blade drive system;
Fig. 16 is a side view of the blade drive system;
Fig. 17 is a perspective view of the blade drive system; and
Fig. 18 is a section view and a top plan view of an alternate blade tube section used in the medical device.

### DETAILED DESCRIPTION

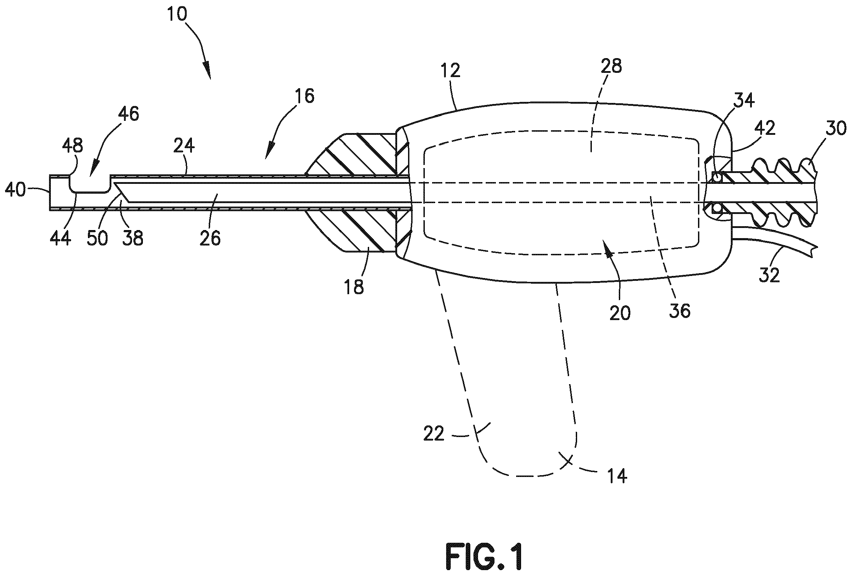

Referring to Fig. 1, there is shown a side view with cut-away sections of a medical device 10 incorporating features of the invention. Although the invention will be described with reference to the exemplary embodiments shown in the drawings, it should be understood that the invention can be embodied in many alternate forms of embodiments. In addition, any suitable size, shape or type of elements or materials could be used.

According to various exemplary embodiments, the medical device 10 is generally configured for use in the removal of nasal polyps, sub-mucosal debulk of turbinates, and functional endoscopic sinus surgery (FESS).

The medical device 10, which may be a disposable debrider for example, comprises a housing 12 (which may have a pistol grip portion 14), a blade tube section 16, and a nosepiece 18. The nosepiece 18 may be a rotatable nosepiece and is between the housing 12 and the blade tube section 16. However, it should be noted that exemplary embodiments of the medical device may comprise any suitable configuration such as configurations having a nosecone coupled to an outer member (of the housing), or any other suitable curved or straight debrider configuration which may comprise an irrigation feature, for example. The blade tube section 16 of the device 10 can be configured with large and small shaver tubes, depending on anatomy and surgeon preference, and can also be adapted for bipolar or monopolar radio-frequency (RF) power. An external ESG (electrosurgical generator) may supply the RF power, for example.

The housing 12 comprises an interior cavity 20 sized and shaped to house actuation members of the device 10. Additionally, in some embodiments the optional pistol grip portion 14 may include an interior cavity 22 which can also be sized and shaped to house actuation members (or other hardware) of the device 10.

The blade tube section 16 comprises an outer blade tube 24 and an inner blade tube 26, and the medical device 10 further comprises a blade drive system 28 mounted within the cavity 20 (or mounted within the cavity 22) which is configured to drive the inner blade tube 26. It should be noted that in some embodiments, the blade tubes 24, 26 may comprise flexible and/or curved tubes.

Additionally, the medical device 10 comprises a connector 30 and a power cable 32. The connector (or suction connection) 30 is configured to connect to a suction tube or a vacuum source. The connector 30 includes a dynamic seal 34 mounted inside of the connector 30. The dynamic seal 34 is configured to provide a sealed interface between the connector 30 and an inner lumen 36 (via the outer surface of the inner blade tube 26) of the inner blade tube 26. The power cable 32 is configured to provide power to components(s) of the blade drive system 28.

The outer blade tube 24 is (rotatably or fixedly) mounted to the housing 12 and acts as a static member. For example, according to various exemplary embodiments, the nosepiece 18 can be mounted to the outer blade tube 24 and can optionally rotate the outer blade tube 24 independent of the housing 12. The inner blade tube 26 is slidably mounted inside the outer blade tube 24 (such that the inner blade tube 26 is slideably mounted within a lumen 38 of the outer blade tube 24).

The inner blade tube 26 is configured to be forced distally [i.e. towards the distal end 40] or proximally [i.e. towards the proximal end 42] by the blade drive system 28. The outer blade tube 24 comprises an opening 44 proximate the distal end 40 which forms a cutting window 46 for the medical device 10. The cutting window 46 is formed by a cutting edge 48 of the outer blade tube (i.e. the distal edge of the opening 44) and a cutting tip 50 of the inner blade tube 26. The reciprocal motion of the inner blade tube 26 provides for the cutting tip 50 to reciprocate relative to the cutting edge 48 to perform tissue cuts (i.e. by bringing the cutting tip 50 into alignment and out of alignment with the opening 44 of the outer blade tube 24). In the embodiment shown in Fig. 1, the cutting edge 48 is at the cylindrical face portion of the cutting window 46. However in alternate embodiments, the cutting edge may be provided at any suitable location along the distal end 40.

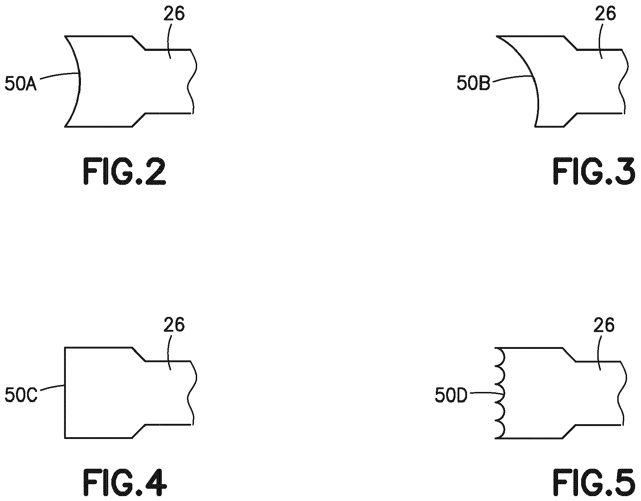

It should be noted that although various exemplary embodiments of the invention have been described in connection with the cutting tip 50 comprising an angled straight edge configuration, alternate embodiments may comprise other suitable configurations. For example, Figs. 2-5 illustrate alternate embodiments for the cutting tip 50 (see cutting tips 50A, 50B, 50C, 50D).

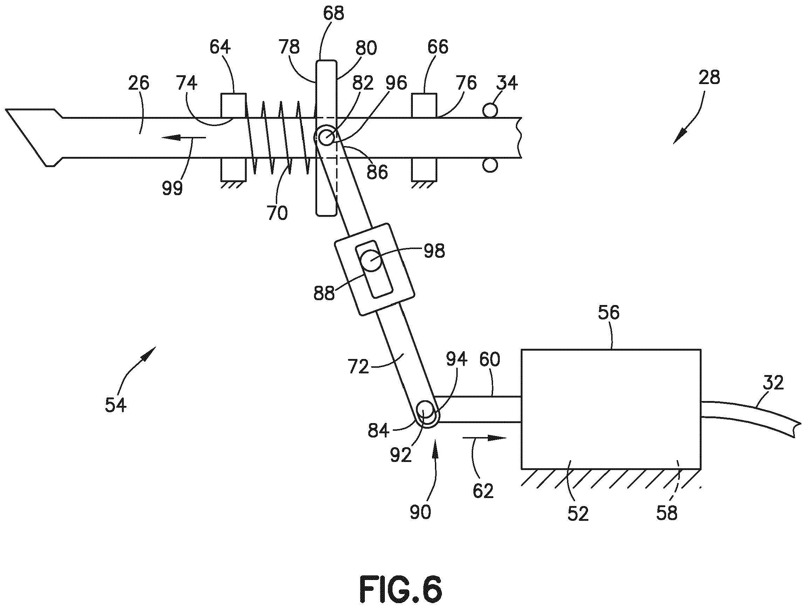

Referring now also to Fig. 6, the blade drive system 28 comprises a solenoid 52 and a mechanical arrangement 54. The solenoid 52 is offset from a central axis of the blade tube section 16. The solenoid 52 generally comprises a static hollow cylindrical section 56 with an energizing coil 58, and a shaft 60 (connected to an armature in the center of the hollow static section 56) that is inducted to move axially (see direction 62) with a known force when the coil 58 is electrically energized (by power cable 32).

The mechanical arrangement 54 comprises a distal bushing 64, a proximal bushing 66, a collar 68, a spring 70, and a lever member 72. The bushings 64, 66 are fixedly mounted to the housing 12 and each comprise an opening 74, 76 sized and shaped to allow reciprocation of the inner blade tube 26 therethrough. The collar 68 comprises a general flange shape and has a first side 78 and a second side 80 and is between the bushings 64, 66. The collar 68 further comprises a pin (or boss feature) 82 between the first and second sides 78, 80. The collar 68 is also fixedly connected (or fixedly mounted) to the inner blade tube 26. The spring 70 is between the distal bushing 64 and the first side 78 of the collar 68. According to various exemplary embodiments, the spring is a compression spring, however in alternate embodiments the spring may be an extension spring. Additionally, in some further embodiments, the spring may be between the proximate bushing 66 and the second side 80 of the collar 68.

The lever member 72 comprises a first end 84, an opposite second end 86, and an opening 88 between the first end 84 and the second end 86. The first end 84 of the lever member is movably connected to the shaft 60 of the solenoid 52 at mount point 90. According to various exemplary embodiments, the mount point 90 comprises a pin (or boss feature) 92 on the shaft 60 and an opening 94 (which receives the pin [or boss feature]) at the first end 84 of the lever member 72. In this embodiment, the opening 94 comprises an elongated or slotted shape to provide a movable connection between the opening 94 and the pin 92. However, in alternate embodiments any other suitable feature, which allows for a movable connection between the lever member and the shaft can be provided. According to various exemplary embodiments, the mount point can be fixed to push & pull, 'push' only, or 'pull' only, depending on the direction of the power stroke of the solenoid and/or the solenoid configuration. The second end 86 of the lever member 72 comprises an opening 96 sized and shaped to receive the pin 82 of the collar 68 which together provides for a movable connection between the lever member 72 and the collar 68. However, in alternate embodiments any other suitable feature, which allows for a movable connection between the lever member and the collar can be provided. According to some embodiments, the opening 96 comprises an elongated or slotted shape.

The opening 88 is sized and shaped to receive a pin (or boss feature) 98 fixedly mounted to the housing 12. According to some embodiments, the opening 88 comprises an elongated or slotted shape. The movable connection between the opening 88 and the pin 98 allows for the lever member 72 to pivot (or rotate) about the pin 98.

The mechanical arrangement described above is configured to have the lever member 72 rotate (and/or slide) about the fixed pin 98 to transfer solenoid actuation to the inner blade tube 26. According to various exemplary embodiments, the 'reverse' solenoid motion (i.e. power stroke of solenoid 52 in direction 62) creates a forward motion (see arrow 99) of the inner blade tube 26. For example, when the solenoid 52 is actuated, the shaft 60 moves in direction 62, which causes the lever arm 72 to pivot or rotate in a counter clockwise direction, which further causes the inner blade tube 26 to move in direction 99. The compression spring 70 is then configured to provide a biasing force in the opposite direction (i.e. opposite of direction 99) to move the inner blade tube 26 (in the direction opposite of 99) and cause a clockwise pivot or rotation of the lever member 72, which returns the shaft 60 of the solenoid 52 in the reverse stroke (i.e. moves the shaft 60 in the direction opposite of 62). This movement of the inner blade tube 26 in the direction 99 and opposite of the direction 99 provides reciprocating motion of the inner blade tube within the outer blade tube.

Although the embodiment above has been described in connection with a 'reverse' solenoid motion (i.e. power stroke of solenoid 52 in direction 62), alternate embodiments may be provided with a power stroke in the opposite direction and an extension spring (in place of the compression spring 70), or a compression spring between the collar 68 and the proximal bushing 66.

It should be noted that in various exemplary embodiments, the pin 98 can be placed in different locations to create different stroke lengths and mechanical force efficiencies relative to the stroke and force of the solenoid. Additionally, it should further be noted that in this embodiment the openings 88, 94 have been described as having a slotted or elongated shape to provide "de-constrained" connections to pins 98, 92 while the connection between pin 82 and opening 96 provides for a substantially fixed pivot point. With this configuration the lever member 72 provides a swinging arc and a single fixed pivot point (at the movable connection between the pin 82 and the opening 96) and "de-constrained" connections (which move in x and y directions relative to the swinging arc [via the elongated/slotted openings 88, 94]) at pins 98, 92. However in alternate embodiments, the fixed pivot point could instead be provided at pin 98 or 92 with the "de-constrained" connections at the remaining two openings 88, 94, 96.

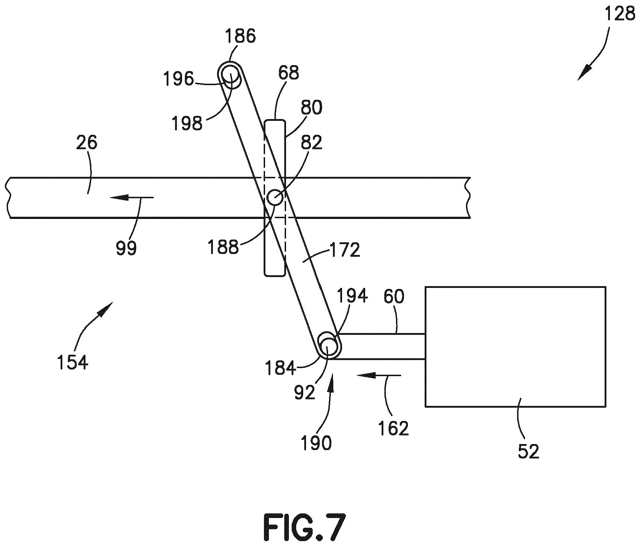

Referring now also to Fig. 7, an alternate embodiment of a blade drive system 128 is shown. The blade drive system 128 is similar to the blade drive system 28 and similar features are similarly numbered. Similar to the mechanical arrangement 54, the mechanical arrangement 154 comprises a lever member 172 having a first end 184, an opposite second end 186, and an opening 188 between the first end 184 and the second end 186. Also similarly the first end 184 of the lever member is movably connected to the shaft 60 of the solenoid 52 at mount point 190 comprising the pin (or boss feature) 92 on the shaft 60 and an opening 194 (which receives the pin [or boss feature]) at the first end 184 of the lever member 172. The opening 194 comprises an elongated or slotted shape to provide a movable connection between the opening 194 and the pin 92. However, in this embodiment, the second end 186 of the lever member 172 comprises an opening 196 sized and shaped to receive a pin 198 which is fixedly mounted to the housing 12. The movable connection between the opening 196 and the pin 198 allows for the lever member 172 to pivot (or rotate) about the pin 198.

Another difference in this embodiment is that the opening 188 is sized and shaped to receive the pin (or boss feature) 82 of the collar 68. According to some embodiments, the opening 188 comprises an elongated or slotted shape.

The mechanical arrangement 154 described above is configured to have the lever member 172 rotate (and/or slide) about the fixed pin 198 to transfer solenoid actuation to the inner blade tube 26. According to various exemplary embodiments, the solenoid motion (i.e. power stroke of solenoid 52 in direction 162) creates a forward motion (see arrow 99) of the inner blade tube 26. For example, when the solenoid 52 is actuated, the shaft 60 moves in direction 162, which causes the lever arm 172 to pivot or rotate about the pin 198, which further causes the collar 68 and the inner blade tube 26 to move in direction 99.

Similar to the mechanical arrangement 54, the mechanical arrangement 154 may comprise compression and/or expansion springs (between the collar and a bushing) to provide a biasing force for the reverse stroke. Additionally, in some other embodiments, the shaft 60 of the solenoid 52 could push directly on the collar 68. Also similar to the embodiment shown in Fig. 6, the openings 188, 194 have been described as having a slotted or elongated shape to provide "de-constrained" connections to pins 82, 92 while the connection between pin 198 and opening 196 provides for a substantially fixed pivot point. With this configuration the lever member 172 provides a swinging arc and a single fixed pivot point (at the movable connection between the pin 198 and the opening 196) and "de-constrained" connections (which move in x and y directions relative to the swinging arc [via the elongated/slotted openings 188, 194]) at pins 82, 92. However in alternate embodiments, the fixed pivot point could instead be provided at pin 82 or 92 with the "de-constrained" connections at the remaining two openings 188, 194, 196.

Technical effects of any one or more of the exemplary embodiments provide significant advantages over conventional configurations by providing greater force to the reciprocating blade than conventional air pressure-based devices can provide. A further technical effect of the various exemplary embodiments is providing a solenoid offset from the main blade to drive the blade directly by way of a flange affixed to the blade, or else indirectly by way of a rigid member on a pivot that reverses the direction of the force. Another technical effect of the various exemplary embodiments is that the blade is mounted with a spring in such a way that after each solenoid movement, the blade returns to its home position.

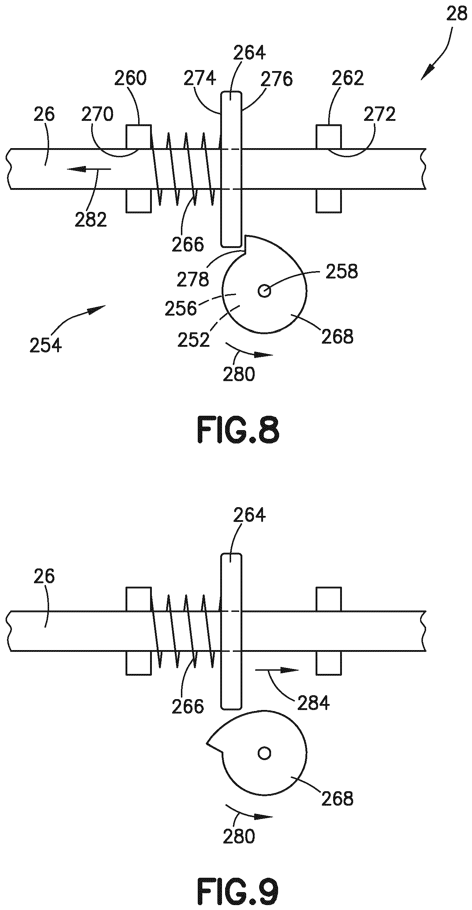

Referring now also to Fig. 8, the blade drive system 28 comprises a motor 252 and a mechanical arrangement 254. The motor 252 is offset and substantially perpendicular relative to a central axis of the blade tube section 16. The motor 252 generally comprises a motor housing 256 and a motor shaft 258 extending from the motor housing 256. The motor shaft 258 is configured to rotate when the motor 252 is electrically energized (by power cable 32). According to some embodiments, the motor 252, or at least a portion of the motor 252, may be disposed within the grip cavity 22. However, in alternate embodiments, any suitable location for the motor 252 may be provided (such as the housing cavity 20, for example).

The mechanical arrangement 254 comprises a distal bushing 260, a proximal bushing 262, a collar 264, a spring 266, and a flywheel 268. The bushings 260, 262 are fixedly mounted to the housing 12 and each comprise an opening 270, 272 sized and shaped to allow reciprocation of the inner blade tube 26 therethrough. The collar 264 comprises a general flange shape and has a first side 274 and a second side 276 and is between the bushings 260, 262. The collar 264 is fixedly connected (or fixedly mounted) to the inner blade tube 26. The spring 266 is between the distal bushing 260 and the first side 274 of the collar 64.

According to various exemplary embodiments, the spring is a compression spring, however in alternate embodiments the spring may be an extension spring. Additionally, in some further embodiments, the spring may be between the proximate bushing 262 and the second side 276 of the collar 264.

The flywheel 268 comprises a cam portion 278. The cam portion 278 forms a projection on the rotating flywheel 268. The cam portion 278 is configured to make sliding contact with the second side 276 of the collar 264 (while the flywheel 268 is rotating) to impart motion to the inner blade tube 26 (through the collar 264 which is fixedly mounted to the inner blade tube 26).

The mechanical arrangement described above is configured to have the flywheel 268 (driven by the motor 252) rotate (see arrow 280) such that the cam portion (or cam shape) 278 interfaces with the collar 264. As the cam portion 278 rotates and engages the second side 276 of the collar 264, rotation of the flywheel 268 causes the cam portion 278 to push, or impart a force, in direction 282 to cause the collar 264 and the inner blade tube 26 to slide towards the distal end 40 (see Fig. 8) . As the cam portion 278 moves past the collar 264 and disengages (see Fig. 9), the inner blade tube is returned to its home position (see arrow 284) due to a biasing force of the spring 266.

Although the embodiment above has been described in connection with a 'counter clockwise' rotation of the flywheel, alternate embodiments may be provided with a 'clockwise rotation' of the flywheel (with a corresponding cam portion) and the spring in the same location or with the spring between the collar 264 and the proximal bushing 262. In further embodiments, any suitable location(s) for the flywheel and/or spring which provide for reciprocal motion of the inner blade tube may be provided.

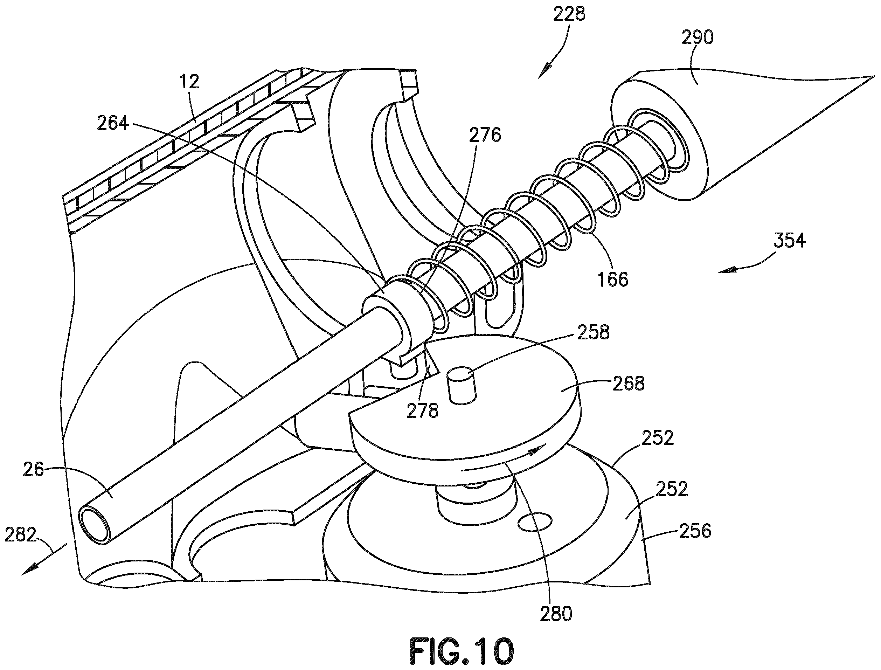

Referring now also to Fig. 10, an alternate embodiment of a blade drive system is shown. The blade drive system 228 is similar to the blade drive system 28 and similar features are similarly numbered. Similar to the mechanical arrangement 254, the mechanical arrangement 354 comprises a collar 264, a spring 166, and a flywheel 268. However, in this embodiment, the spring 166 is an extension spring connected between the collar 264 and a suction connector 290.

Similar to the blade drive system 28, the cam portion 278 is configured to make sliding contact with the second side 276 of the collar 264 (while the flywheel 268 is rotating) to impart motion to the inner blade tube 26 (through the collar 264 which is fixedly mounted to the inner blade tube 26). The mechanical arrangement 354 is configured to have the flywheel 268 (driven by the motor 252) rotate (see arrow 280) such that the cam portion (or cam shape) 278 interfaces with the collar 264. As the cam portion 278 rotates and engages the second side 276 of the collar 264, rotation of the flywheel 268 causes the cam portion 278 to push, or impart a force, in direction 282 to cause the collar 264 and the inner blade tube 26 to slide towards the distal end 40. As the cam portion 278 moves past the collar 264 and disengages, the inner blade tube is returned to its home position due to a biasing force of the extension spring 166. It should be noted that although this embodiment shows the spring 166 connected to the suction member 166, alternate embodiments may have the spring 166 connected to any other suitable fixed surface of e the device 10.

Technical effects of any one or more of the exemplary embodiments provide significant advantages over conventional configurations by providing greater force to the reciprocating blade than conventional air pressure-based devices can provide. A further technical effect of the various exemplary embodiments is providing a motor offset (and perpendicular) from a central axis of the blade tube section. Another technical effect of the various exemplary embodiments is that the blade is mounted with a spring in such a way that after each movement, the blade returns to its home position.

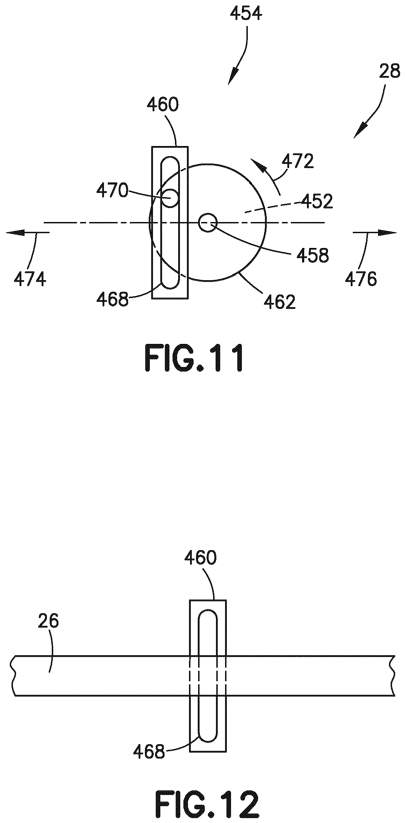

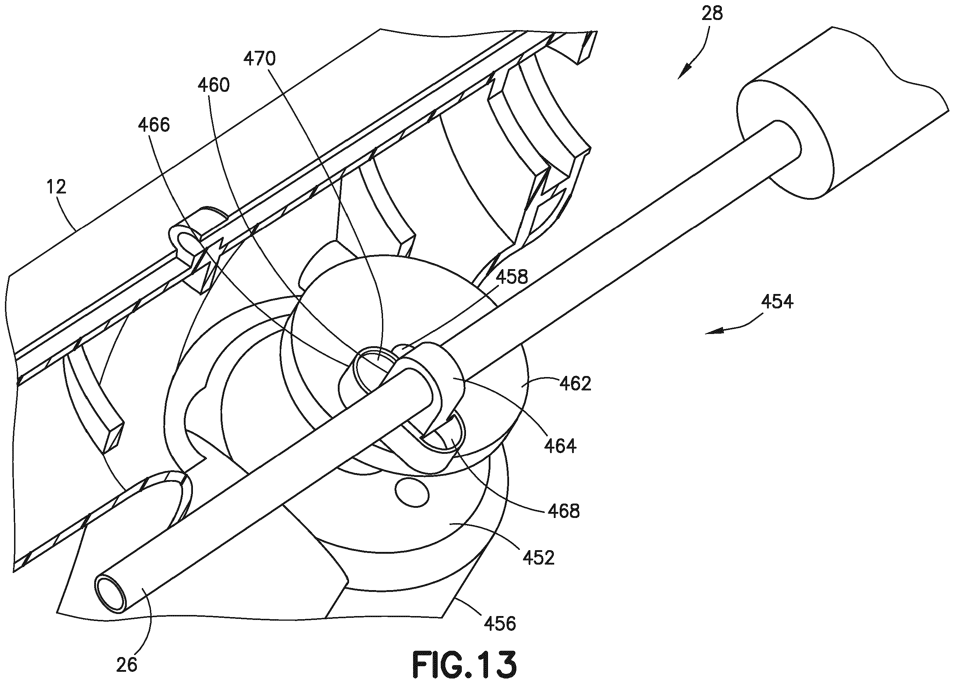

Referring now also to Fig. 11-13, the blade drive system 28 comprises a motor 452 and a mechanical arrangement 454. The motor 452 is substantially perpendicular relative to a central axis of the blade tube section 16. The motor 452 generally comprises a motor housing 456 and a motor shaft 458 extending from the motor housing 456. The motor shaft 458 is configured to rotate when the motor 452 is electrically energized (by power cable 32). According to some embodiments, the motor 452, or at least a portion of the motor 452, may be disposed within the grip cavity 22. However, in alternate embodiments, any suitable location for the motor 452 may be provided (such as the housing cavity 20, for example).

The mechanical arrangement 454 comprises a collar 460 and a flywheel 462. The collar 460 comprises a flange section 464 and a yoke section 466. The flange section 464 is fixedly connected (or fixedly mounted) to the inner blade tube 426. The yoke section 466 comprises a slotted portion 468 having a general slot or racetrack shape. The flywheel comprises a pin 470 configured to be received in the slotted portion 468. The configuration of the yoke section 466 of the collar 460 and the pin 470 of the flywheel 462 forms a "scotch yoke" or a "slotted link mechanism" providing a reciprocating motion mechanism which converts the rotational motion of the flywheel 462 into linear motion of the collar 460 (and inner blade tube 26). For example, as the flywheel 462 rotates in direction 472, the pin 470 imparts a force on the collar 460 (in direction 474) while the pin 470 slides within the slotted portion 468. As the flywheel 462 continues to rotate, the collar 460 (and inner blade tube 26) reaches the end of the forward stroke, the continued rotation of the flywheel 462 begins to cause the pin 470 to impart a force on the collar 460 (in direction 476) while the pin 470 continues to slide within the slotted portion 468. As the flywheel 462 continues to rotate, the collar 460 (and inner blade tube 26) reaches the end of the reverse stroke and the continued rotation of the flywheel 462 begins to cause the pin 470 to impart a force on the collar 460 in the direction 476 to repeat the forward stroke.

In the embodiments described above the mechanical arrangement comprises the pin 470 on the flywheel 462 and the slotted portion 468 on the yoke section 466 of the collar. However, in alternate embodiments, the pin could be provided on the collar with a slotted section or portion (configured to receive the pin) on the flywheel. Additionally, in other alternate embodiments, any suitable combination of pin and slot features between the driving/flywheel and driven/collar components could be provided. It should further be noted that in various exemplary embodiments the diameter of the flywheel 462 (and related position of the drive pin 470) can be changed to create more/less drive force and more/less actuation stroke (this would have an inverse relationship [more stroke/less force, etc.]).

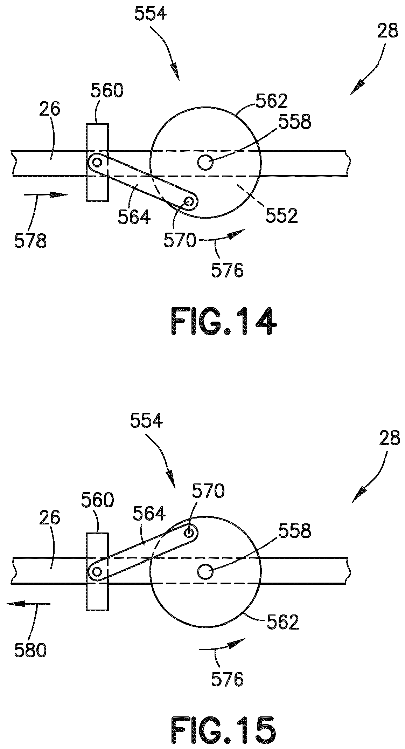

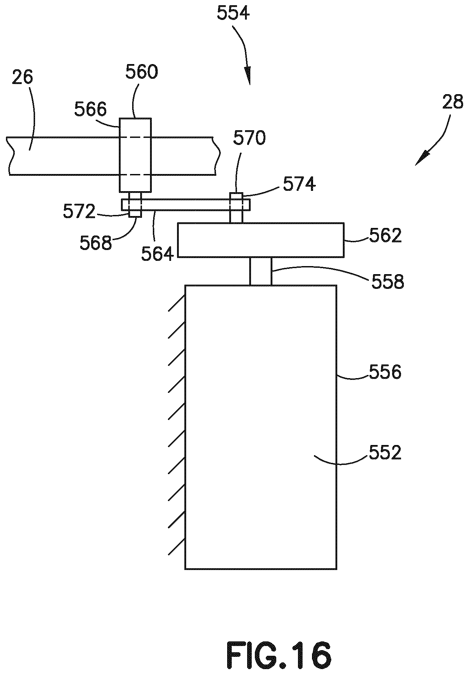

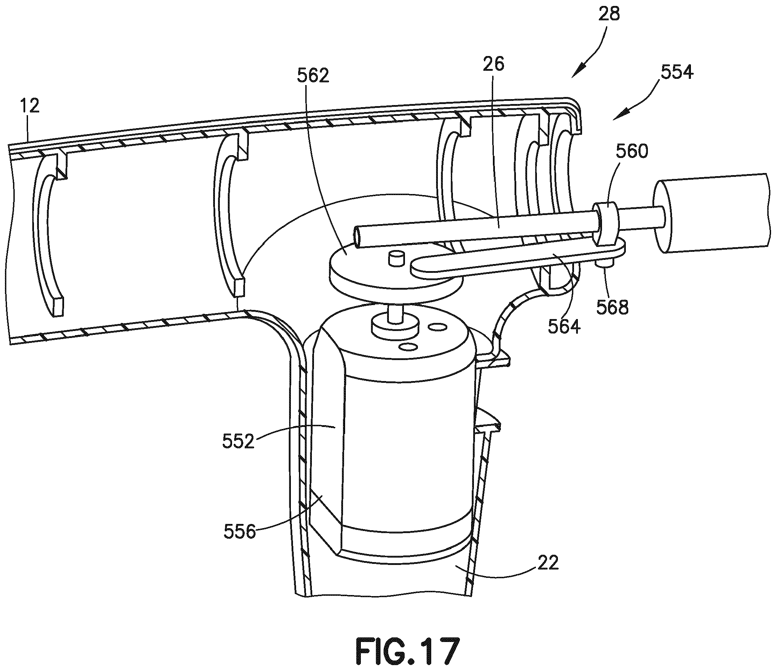

Referring now also to Fig. 14-17, the blade drive system 28 comprises a motor 552 and a mechanical arrangement 554. The motor 552 is substantially perpendicular relative to a central axis of the blade tube section 16. The motor 552 generally comprises a motor housing 556 and a motor shaft 558 extending from the motor housing 556. The motor shaft 558 is configured to rotate when the motor 552 is electrically energized (by power cable 32). According to some embodiments, the motor 552, or at least a portion of the motor 552, may be disposed within the grip cavity 22. However, in alternate embodiments, any suitable location for the motor 552 may be provided (such as the housing cavity 20, for example).

The mechanical arrangement 554 comprises a collar 560, a flywheel 562, and a lever arm 564 between the collar and the flywheel. The collar 560 comprises a flange section 566 and a pin section 568. The flange section 566 is fixedly connected (or fixedly mounted) to the inner blade tube 26. The pin section 568 comprises a general pin shape. The flywheel comprises a pin 570. The lever arm 564 comprises openings 572, 574. The opening 572, 574 are configured to receive the pins 568, 570, respectively.

The mechanical arrangement 554 provides a flywheel with a pin and a rigid member (lever arm) to transform rotational movement from a rotating motor into reciprocating motion. For example, in a locomotive steam engine, the hot steam is pumped into the chambers which causes the piston to move in a reciprocating manner, which drives the linkages to cause the wheels to rotate on the train. The mechanical arrangement 554 is a similar assembly, however the wheel is directly turned, causing the reciprocating as a result. The reciprocating end is connected to the inner blade tube, causing it to reciprocate in turn.

For example, as the flywheel 562 rotates in direction 576 (see Fig. 14), the pin 570 imparts a force on the collar 560 (through the lever arm 564) in direction 578 while the pin 570 rotates within the opening 574. As the flywheel 562 continues to rotate, the collar 560 (and inner blade tube 26) reaches the end of the reverse stroke, the continued rotation of the flywheel 562 begins to cause the pin 570 to impart a force on the collar 560 (through the lever arm 564) in direction 580 while the pin 570 continues to rotate within the opening 574 (see Fig. 15). As the flywheel 562 continues to rotate, the collar 560 (and inner blade tube 26) reaches the end of the forward stroke and the continued rotation of the flywheel 562 begins to cause the pin 570 to impart a force on the collar 560 in the direction 578 to repeat the reverse stroke.

Although the embodiment above has been described in connection with a 'counter clockwise' rotation of the flywheel, alternate embodiments may be provided with a 'clockwise rotation' of the flywheel to provide for reciprocal motion of the inner blade tube.

Technical effects of any one or more of the exemplary embodiments provide significant advantages over conventional configurations by providing greater force to the reciprocating blade than conventional air pressure-based devices can provide. A further technical effect of the various exemplary embodiments is providing a motor perpendicular from a central axis of the blade tube section.

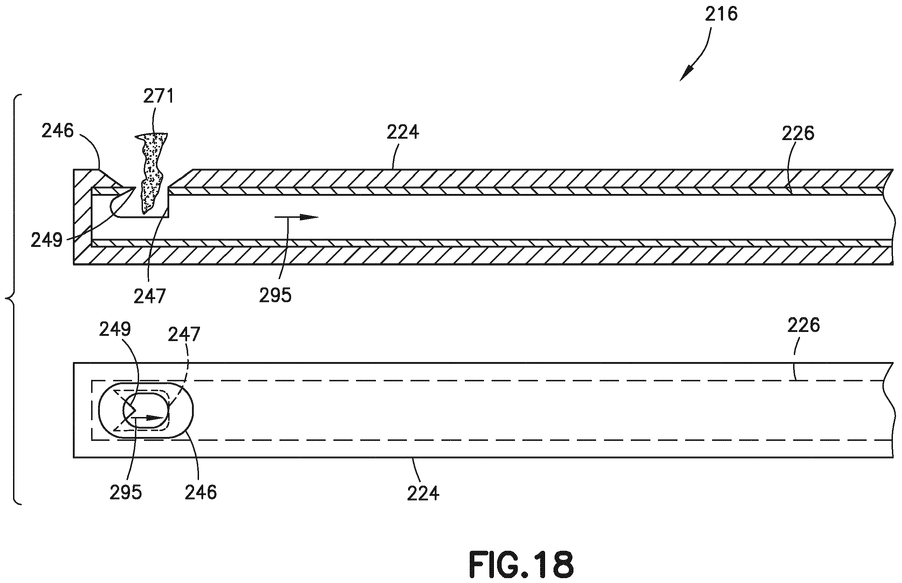

While various exemplary embodiments of the invention have been described in connection with a blade tube section 16 having a cutting edge 48 at the cutting window 46 of the outer blade tube 24, other configurations may be provided. For example, an alternate embodiment of a blade tube section 216 is shown in Fig. 18 (illustrating a cross-section view [top] and a top plan view [bottom]). Similar to the blade tube section 16, the blade tube section 216 comprises an outer blade tube 224 and an inner blade tube 226 configured to be driven by the blade drive system 28. However, in this embodiment, the inner blade tube 226 comprises a cutting window 247 and a cutting edge 249. The cutting window 247 is configured to be aligned with the window 246 of the outer blade tube 224 such that the cutting is provided when tissue 271 extends through the windows 246, 247 and a backwards motion of the inner blade tube 226 (towards the proximate end [see arrow 295]) causes the cutting edge 249 to cut through the tissue 271.

Below are provided further descriptions of various non-limiting, exemplary embodiments. The below-described exemplary embodiments may be practiced in conjunction with one or more other aspects or exemplary embodiments. That is, the exemplary embodiments of the invention, such as those described immediately below, may be implemented, practiced or utilized in any combination (e.g., any combination that is suitable, practicable and/or feasible) and are not limited only to those combinations described herein and/or included in the appended claims.

In one example embodiment, a medical device is provided comprising: a blade tube section comprising an outer blade tube, an inner blade tube, and a cutting window at a distal end of the blade tube section; a solenoid offset from a central axis of the blade tube section; and a mechanical arrangement between the inner blade tube and the solenoid.

The mechanical arrangement may comprise a lever member, wherein the lever member is connected to the inner blade tube and the solenoid. The medical device may further comprise a collar connected to the inner tube member. The lever member may be movably connected to the collar. The mechanical arrangement may be configured such that movement of a shaft of the solenoid in a first direction provides for movement of the inner blade tube in a second opposite direction. The mechanical arrangement may be configured such that movement of a shaft of the solenoid in a first direction provides for movement of the inner blade tube in the same direction. The medical device may further comprisie a collar, a bushing, and a spring, wherein the collar is connected to the inner tube member, and wherein the spring is between the bushing and the collar. The inner blade tube may be configured to reciprocate relative to the outer blade tube.

In another example embodiment, a medical device is provided comprising: a blade tube section comprising an outer blade tube, an inner blade tube, and a cutting window at a distal end of the blade tube section; a solenoid; and a lever member connected to the inner blade tube and the solenoid.

A mechanical arrangement may be between the inner blade tube and the solenoid. The medical device may further comprise a collar fixedly connected to the inner tube member. The lever member may be movably connected to the collar. The lever member may comprise a first end, a second end, and an opening between the first end and the second end, wherein the lever member is configured to rotate about the opening. The lever member may comprise a first end, a second end, and an opening between the first end and the second end, wherein the lever member is configured to rotate about the second end. The medical device may further comprise a collar, a bushing, and a spring, wherein the collar is connected to the inner tube member, and wherein the spring is between the bushing and the collar. The inner blade tube may be configured to reciprocate relative to the outer blade tube. The solenoid may be offset from a central axis of the blade tube section.

In another example embodiment, a medical device is provided comprising: a blade tube section comprising an outer blade tube, an inner blade tube, and a cutting window at a distal end of the blade tube section; a motor offset from a central axis of the blade tube section; and a mechanical arrangement between the inner blade tube and the motor.

The medical device may further comprise a collar connected to the inner tube member. The mechanical arrangement may comprise a flywheel, wherein the flywheel is connected to the motor. The flywheel may comprise a cam portion. The cam portion may be configured to engage the collar. The motor may comprise a motor shaft, wherein the motor shaft is substantially perpendicular to the central axis of the blade tube section. The medical device may further comprise a collar, a bushing, and a spring, wherein the collar is connected to the inner tube member, and wherein the spring is between the bushing and the collar. The inner blade tube may be configured to reciprocate relative to the outer blade tube.

In another example embodiment, a medical device is provided comprising: a blade tube section comprising an outer blade tube, and inner blade tube, and a cutting window at a distal end of the blade tube section; a motor; and a cam portion between the inner blade tube and the motor.

The medical device may further comprise a collar fixedly connected to the inner tube member. A mechanical arrangement may be provided between the inner blade tube and the motor. The mechanical arrangement may comprise a flywheel, wherein the flywheel is connected to the motor. The flywheel may comprise a cam portion. The cam portion may be configured to contact a member on the inner blade tube. The medical device may further comprise a collar, a bushing, and a spring, wherein the collar is connected to the inner tube member, and wherein the spring is between the bushing and the collar. The inner blade tube may be configured to reciprocate relative to the outer blade tube. The motor may be substantially perpendicular from a central axis of the blade tube section.

In another example embodiment, a medical device is provided comprising: a blade tube section comprising an outer blade tube, an inner blade tube, and a cutting window at a distal end of the blade tube section; a motor spaced from the blade tube section; and a scotch yoke or slotted link mechanism between the inner blade tube and the motor.

The medical device may further comprise a collar connected to the inner tube member. The medical device may further comprise a flywheel, wherein the flywheel is connected to the motor. The flywheel may comprise a pin. The pin may be configured to engage a portion of the collar. The motor may comprise a motor shaft, wherein the motor shaft is substantially perpendicular to the central axis of the blade tube section. The medical device may further comprise a collar, wherein the collar is connected to the inner tube member, and wherein a yoke portion of the collar is configured to receive a pin rotated by the motor. The inner blade tube may be configured to reciprocate relative to the outer blade tube.

In another example embodiment, a medical device is provided comprising: a blade tube section comprising an outer blade tube, an inner blade tube, and a cutting window at a distal end of the blade tube section; a motor; a flywheel having a pin, wherein the flywheel is connected to the motor; and a slotted portion between the inner blade tube and the motor, wherein the slotted portion is configured to receive the pin.

The medical device may further comprise a collar fixedly connected to the inner tube member. A mechanical arrangement may be between the inner blade tube and the motor. The mechanical arrangement may comprise the flywheel. The flywheel may comprise a pin. The pin is configured to engage a portion of the collar. The medical device may further comprise a collar, wherein the collar is connected to the inner tube member, and wherein a yoke section of the collar comprises the slotted portion. The inner blade tube may be configured to reciprocate relative to the outer blade tube. The motor may be substantially perpendicular relative to a central axis of the blade tube section.

In another example embodiment, a medical device is provided comprising: a blade tube section comprising an outer blade tube, an inner blade tube, and a cutting window at a distal end of the blade tube section; a motor spaced from the blade tube section; and a lever arm between the inner blade tube and the motor.

The medical device may further comprise a collar connected to the inner tube member. The medical device may further comprise a flywheel, wherein the flywheel is connected to the motor. The flywheel may comprise a pin. The collar may comprise a flange section and a pin section. The motor may comprise a motor shaft, wherein the motor shaft is substantially perpendicular to the central axis of the blade tube section. The medical device may further comprise a collar, wherein the collar is connected to the inner tube member, and wherein a pin of the collar is configured to be received by an opening of the lever arm. The inner blade tube may be configured to reciprocate relative to the outer blade tube.

In another example embodiment, a medical device is provided comprising: a blade tube section comprising an outer blade tube, an inner blade tube, and a cutting window at a distal end of the blade tube section; a motor; a flywheel having a pin, wherein the flywheel is connected to the motor; and a collar having a pin section, wherein the collar is connected to the inner blade tube, wherein a member movably connects the pin section to the pin.

The collar may be fixedly connected to the inner tube member. The member may be a lever arm. The lever arm may comprise a first opening and an opposite second opening. The first opening may be configured to receive the pin, and wherein the second opening is configured to receive the pin section. The lever arm may be between the flywheel and the inner blade tube. The pin may be between the flywheel and the inner blade tube. The inner blade tube may be configured to reciprocate relative to the outer blade tube. The motor may be substantially perpendicular relative to a central axis of the blade tube section.

It should be understood that components of the invention can be operationally coupled or connected and that any number or combination of intervening elements can exist (including no intervening elements). The connections can be direct or indirect and additionally there can merely be a functional relationship between components.

It should be understood that the foregoing description is only illustrative of the invention. Various alternatives and modifications can be devised by those skilled in the art without departing from the invention. Accordingly, the invention is intended to embrace all such alternatives, modifications and variances which fall within the scope of the appended claims.

## Claims

1. A medical device comprising:
a blade tube section comprising an outer blade tube, an inner blade tube, and a cutting window at a distal end of the blade tube section;
a blade drive system; and
a mechanical arrangement between the inner blade tube and the blade drive system.

2. The medical device of claim 1, wherein the blade drive system includes a solenoid.

3. The medical device of claim 2, wherein the solenoid is offset from a central axis of the blade tube section.

4. The medical device as in any of claim 1-3, wherein the mechanical arrangement comprises a lever member, wherein the lever member is connected to the inner blade tube and the blade drive system.

5. The medical device of any of claims 1 to 4, wherein the mechanical arrangement comprises a collar connected to the inner tube member.

6. The medical device of claim 5, wherein the lever member is movably connected to the collar.

7. The medical device of any of claims 2 to 6, wherein the mechanical arrangement is configured such that movement of a shaft of the solenoid in a first direction provides for movement of the inner blade tube in a second opposite direction.

8. The medical device of any of claims 2 to 6, wherein the mechanical arrangement is configured such that movement of a shaft of the solenoid in a first direction provides for movement of the inner blade tube in the same direction.

9. The medical device of any of claims 4 to 8, wherein the lever member comprises a first end, a second end, and an opening between the first end and the second end, wherein the lever member is configured to rotate about the opening.

10. The medical device of any of claims 4 to 8, wherein the lever member comprises a first end, a second end, and an opening between the first end and the second end, wherein the lever member is configured to rotate about the second end.

11. The medical device as in any of claim 1-10, wherein the mechanical arrangement comprises a collar, a bushing, and a spring, wherein the collar is connected to the inner tube member, and wherein the spring is between the bushing and the collar.

12. The medical device as in any of claim 1-11, wherein the inner blade tube is configured to reciprocate relative to the outer blade tube.
